# EUROPEAN PATENT SPECIFICATION

(11) **EP 2 218 760 B1**
(45) Date of publication and mention of the grant of the patent: **02.09.2015**
(21) Application number: 08854633.8
(22) Date of filing: 19.11.2008
(51) Int. Cl.: C09K 3/30, A24B 15/10, A24F 47/00

(54) **AEROSOL-GENERATING SOLUTION FOR AEROSOL ASPIRATOR**
AEROSOLBILDENDE LÖSUNG FÜR EINEN AEROSOLASPIRATOR
SOLUTION DE GÉNÉRATION D'UN AÉROSOL POUR APPAREIL ASPIRATOIRE À AÉROSOL

(30) Priority: 30.11.2007 JP 2007310567
(43) Date of publication of application: 18.08.2010
(73) Proprietor: Japan Tobacco Inc., Tokyo 105-8422 (JP)
(72) Inventor: KATAYAMA, Kazuhiko, Tokyo 130-8603 (JP); YAJIMA, Morio, Tokyo 130-8603 (JP)
(74) Representative: Isarpatent
(86) International application number: PCT/JP2008/071016
(87) International publication number: WO 2009/069519

(56) References cited:
- EP-A1- 1 618 803
- EP-A2- 0 336 456
- JP-A- 2 190 178
- JP-A- 10 179 112
- JP-T- 2001 501 452
- JP-T- 2007 512 880
- JP-T- 2007 532 118
- US-A1- 2007 062 548

## Description

### Technical Field

The present invention relates to aerosol-generating liquids suited for use as an aroma solution for an aerosol inhalator which is configured to generate an aerosol by heating and atomizing the aroma solution when the user inhales, to allow the user to take in the aerosol together with air, in particular, an aerosol inhalator for pseudo smoking.

### Background Art

Aerosol inhalators of this type are disclosed, for example, in PCT-based Japanese Laid-open Patent Publication No. 2000-510763 and Japanese Patents No. 3484233 and No. 3488717. The aerosol inhalators disclosed in these publications use respective different methods for atomizing an aroma to generate an aerosol. Specifically, in the inhalator of PCT-based Japanese Laid-open Patent Publication No. 2000-510763, an aroma solution containing an aroma is heated for atomization. In the inhalator of Japanese Patent No. 3484233, ultrasonic waves are applied to an aroma solution to atomize same, and in the inhalator of Japanese Patent No. 3488717, an aroma solution is sprayed for atomization.

### Disclosure of the Invention

### Problems to be Solved by the Invention

Whichever method of the above three aerosol inhalators is used to atomize the aroma solution, the relative amount of the aromatic component in the generated aerosol, namely, the aerosolizing efficiency of the aromatic component, is low. Accordingly, when the aerosol is inhaled together with air, the user is unable to fully relish the aerosolized aromatic component, that is, the aroma of the aromatic aerosol.

Also, the aromatic aerosol is low in longevity. Thus, where the aromatic aerosol is inhaled for purposes of pseudo smoking, the aromatic aerosol disappears immediately after it is introduced into the user's oral cavity. The user is therefore unable to take his/her time in relishing the aroma of the aromatic aerosol, unlike ordinary cigarette smoking.

EP1618803, EP0336456 and US20070062548 describe aerosol generating compositions comprising volatile organic acids,such as citric acid, levulinic acid and citric acid.

An object of the present invention is therefore to provide an aerosol-generating liquid for use in an aerosol inhalator, which liquid is capable of generating a sufficient amount of aromatic aerosol and also allows the user to take time to relish the aroma of the aromatic aerosol introduced into his/her mouth.

### Means for Solving the Problems

To achieve the object, the present invention provides an aerosol-generating liquid for use in an aerosol inhalator, wherein the aerosol-generating liquid contains main components including a solvent and a lipophilic aroma dissolved in the solvent, and an adipic acid added to the main components

Preferably, the carboxylic acid accounts for 10 weight % or less of the main components.

Where the aerosol-generating liquid is used with an aerosol inhalator and is inhaled in the form of an aromatic aerosol by the user, the carboxylic acid contained in the aerosol-generating liquid ensures that the aerosol-generating liquid exhibits improved aerosolizing efficiency and longevity of the aromatic aerosol, compared with aerosol-generating liquids containing no carboxylic acid.

Specifically, the main components contain propylene glycol as the solvent and L-menthol as the aroma. In this case, the aerosol-generating liquid is suited for pseudo smoking.

### Advantageous Effects of the Invention

Where the aerosol-generating liquid is used for pseudo smoking, the liquid can efficiently generate an aromatic aerosol with high longevity. As a result, the user is allowed to take time to fully relish the flavor and taste of the aromatic aerosol in his/her mouth, so that the user can enjoy pseudo smoking just like ordinary cigarette smoking.

### Brief Description of the Drawings

FIG. 1 is a sectional view of an exemplary aerosol inhalator used for testing; and
FIG. 2 is a graph showing the relationship of transmitted light attenuation rates of aromatic aerosols with elapsed time.

### Best Mode of Carrying out the Invention

An aerosol-generating liquid for use in an aerosol inhalator contains main components and an adipic acid added as an additive to the main components. Specifically, the main components include a solvent and a lipophilic aroma dissolved in the solvent.

It is desirable that the carboxylic acid accounts for 10 weight % or less, preferably, 3 weight % or less of the main components. The adipic acid may be used in combination with 1 or more carboxilic acids selected from the group of acetic acid, tartaric acid, citric acid and lauric acid .

### EXAMPLES

Aerosol-generating liquids A to F explained below were prepared.

All of the aerosol-generating liquids A to F contained, as their main components, propylene glycol as the prime solvent and L-menthol as the lipophilic aroma. The aerosol-generating liquids A to E respectively contained acetic acid, tartaric acid, adipic acid, citric acid and lauric acid as the carboxylic acid, while the aerosol-generating liquid F contained only the aforementioned main components.

Table 1 below shows the respective compositions of the aerosol-generating liquids A to F.

**Table 1**

| | A | B | C | D | E | F |
|---|---|---|---|---|---|---|
| Propylene glycol (wt%) | 65 | 65 | 72 | 65 | 65 | 75 |
| L-menthol (wt%) | 25 | 25 | 25 | 25 | 25 | 25 |
| Acetic acid (wt%) | 10 | 0 | 0 | 0 | 0 | 0 |
| Tartaric acid (wt%) | 0 | 10 | 0 | 0 | 0 | 0 |
| Adipic acid (wt%) | 0 | 0 | 3 | 0 | 0 | 0 |
| Citric acid (wt%) | 0 | 0 | 0 | 10 | 0 | 0 |
| Lauric acid (wt%) | 0 | 0 | 0 | 0 | 10 | 0 |

With respect to the above aerosol-generating liquids A to F, the following tests 1 to 3 were conducted for the purpose of comparing the aroma aerosolizing efficiencies of the respective liquids, as well as the longevities, flavors and tastes of respective aromatic aerosols.

### TEST 1

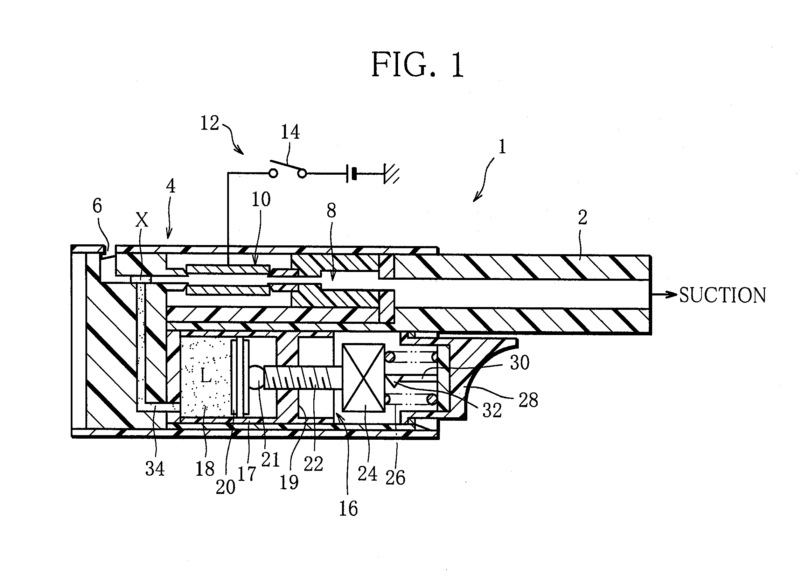

Using an aerosol inhalator shown in FIG. 1, each of the aerosol-generating liquids C and F was aerosolized with a predetermined amount (e.g., 2 mg) of the liquid fed at a time, and the aromatic aerosol delivered from the mouthpiece of the inhalator was collected by means of a filter. Subsequently, the ratios of the amounts of the aerosol-generating liquids C and F collected by the filter to the feed amounts of the respective liquids C and F, that is, the collection efficiencies of the aerosol-generating liquids C and F, were obtained. The results are shown in Table 2 below.

**Table 2**

| | C | F |
|---|---|---|
| Collection efficiency | 72% | 66% |

As is clear from Table 2, the aerosol-generating liquid C containing adipic acid as the carboxylic acid is higher in collection efficiency than the aerosol-generating liquid F containing no adipic acid. This means that the aerosolizing efficiency of the aerosol-generating liquid C, that is, the aerosolizing efficiency of the aroma, is higher than that of the aerosol-generating liquid F.

FIG. 1 illustrates the aerosol inhalator 1 used in conducting the aforementioned Test 1. In the following, the inhalator 1 will be briefly explained.

The inhalator 1 has a casing 4 which includes a mouthpiece 2 projecting from a rear end thereof. The casing 4 further includes an outside air inlet hole 6 formed in the outer surface of a distal end portion thereof and an aerosol generation channel 8 formed therein. The aerosol generation channel 8 extends from the outside air inlet hole 6 to the mouthpiece 2.

Part of the aerosol generation channel 8 is constituted by a tubular electric heater 10 arranged inside the casing 4. The heater 10 is electrically connected to a power supply circuit 12 including a power switch 14. When the power switch 14 is ON, the power supply circuit 12 supplies electric power to the heater 10 to raise the temperature of the heater 10 up to a predetermined temperature.

A cartridge-type syringe pump 16 is accommodated in the casing 4. The syringe pump 16 includes a piston 20 arranged within a syringe barrel 17 thereof, and the piston 20 defines a pump chamber 18 inside the syringe barrel 17. A screw-type piston rod 22 is connected to the piston 20 by means of a ball-and-socket joint 21. The piston rod 22 penetrates through a partition wall 19 of the syringe barrel 17 and is rotatably supported by the partition wall 19.

Further, the piston rod 22 is connected through a rotating cam 24 and a return spring 26 to a push button 28 having a push rod 30. The push rod 30 projects from the push button 28 toward the rotating cam 24 and has a pusher 32 at a distal end thereof. Each time the push button 28, that is, the push rod 30, is pushed and then released, the pusher 32 pushes the piston 20 into the pump chamber 18 by a predetermined distance at a time, in cooperation with the rotating cam 24, thereby decreasing the volume of the pump chamber 18.

A liquid passage 34 extends from the pump chamber 18 toward the aerosol generation channel 8 and meets the channel 8 at a junction X located upstream of the heater 10. Accordingly, when the push button 28 is pushed and released with the pump chamber 18 and the liquid passage 34 filled with a solution L, namely, the aerosol-generating liquid C or F, an amount of the solution L corresponding to the distance over which the piston 20 is moved is supplied to the junction X of the aerosol generation channel 8.

If, at this time, the user draws in the air in the aerosol generation channel 8, that is, takes a puff through the mouthpiece 2, outside air is introduced into the aerosol generation channel 8 from the outside air inlet hole 6, with the result that a suction air flow is produced inside the aerosol generation channel 8. The suction air flow thus produced moves the solution L at the junction X toward the heater 10. On reaching the heater 10, the solution L is heated and atomized by the heater 10, turning into an aromatic aerosol. The aromatic aerosol is then delivered to outside of the mouthpiece 2 together with the suction air.

The amount of the solution L fed at a time to the junction X is 2 mg, as stated above. The mouthpiece 2 of the inhalator 1 is connected to an automatic cigarette smoking machine (not shown), which is configured to perform the aforementioned operation of drawing in the air, namely, a puff.

### TEST 2

Each of the aerosol-generating liquids A to F was filled in the inhalator 1 of FIG. 1, and then using the automatic smoking machine, the inhalator 1 was repeatedly puffed ten times so that the aromatic aerosol may be delivered from the inhalator 1. The aromatic aerosol corresponding in amount to ten puffs was trapped in an airtight chamber. Subsequently, the aromatic aerosol contained in the airtight chamber was irradiated with laser light to measure the intensity of the transmitted laser light, namely, change with time in the attenuation rate of the transmitted laser light.

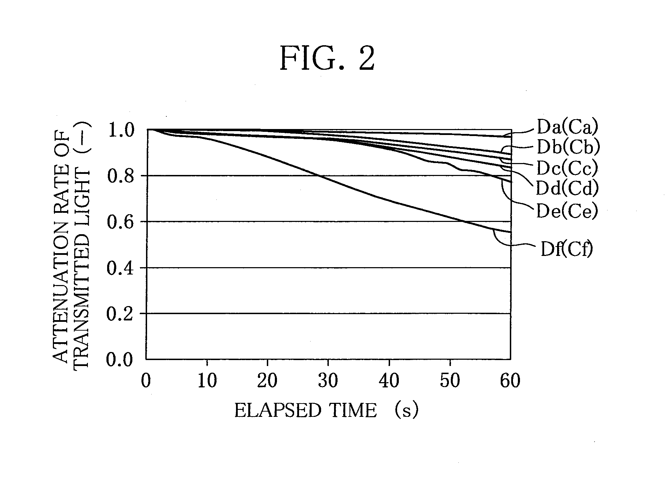

In FIG. 2, Da to Df indicate the respective changes with time in the transmitted light attenuation rate obtained with aromatic aerosols Ca to Cf. The aromatic aerosols Ca to Cf were derived from the aerosol-generating liquids A to F, respectively.

As is clear from FIG. 2, the transmitted light attenuation rates of the aromatic aerosols Ca to Ce are low, compared with that of the aromatic aerosol Cf. This proves that the aromatic aerosols Ca to Ce are higher in longevity than the aromatic aerosol Cf.

### TEST 3

Sensory testers actually inhaled each of the aromatic aerosols Cc and Cf by using the inhalator 1 of FIG. 1, to evaluate the aromatic aerosols Cc and Cf. The evaluation results are as follows:
In the case of the aromatic aerosol Cf, substantial sensory stimulus of L-menthol was perceived in the oral cavity. In the case of the aromatic aerosol Cc, on the other hand, the testers received the sensory stimulus of L-menthol from the oral cavity through to the back of the throat, and also when the aromatic aerosol Cc was exhaled, the sensory stimulus of L-menthol was still perceived. This reveals that the aromatic aerosol Cc obtained from the aerosol-generating liquid C has improved longevity, compared with the aromatic aerosol Cf obtained from the aerosol-generating liquid F.

## Claims

1. An aerosol-generating liquid for use in an aerosol inhalator, the aerosol inhalator being configured to generate an aerosol by heating and atomizing an aroma solution during inhalation and to allow the aerosol to be inhaled together with a suction air flow, wherein the aerosol-generating liquid used as the aroma solution contains:
main components including a solvent and a lipophilic
aroma dissolved in the solvent; and
adipic acid added to the main components.

2. The aerosol-generating liquid according to claim 1, wherein the adipic acid is present in an amount of 3 weight % of the main components.

3. The aerosol-generating liquid according to claim 1, wherein the solvent contains propylene glycol.

4. The aerosol-generating liquid according to claim 1, wherein the aroma contains L-menthol.

5. The aerosol-generating liquid according to claim 1, wherein the main components contain propylene glycol as the solvent and L-menthol as the aroma.

## Patentansprüche

1. Aerosolbildende Flüssigkeit zur Verwendung in einem Aerosolinhalator, wobei der Aerosolinhalator dafür ausgelegt ist, dass ein Aerosol gebildet wird, indem eine Aromalösung während des Inhalierens erhitzt und versprüht wird, und dafür, dass das Aerosol zusammen mit einem Saugluftstrom inhaliert werden kann, wobei die aerosolbildende Flüssigkeit, die als die Aromalösung verwendet wird, Folgendes enthält:
Hauptbestandteile einschließlich eines Lösungsmittels und
eines lipophilen Aromas, das in dem Lösungsmittel gelöst ist, und
Adipinsäure, die den Hauptbestandteilen zugesetzt wurde.

2. Aerosolbildende Flüssigkeit nach Anspruch 1, wobei die Adipinsäure in einer Menge von 3 Gewichts-% der Hauptbestandteile vorliegt.

3. Aerosolbildende Flüssigkeit nach Anspruch 1, wobei das Lösungsmittel Propylenglykol enthält.

4. Aerosolbildende Flüssigkeit nach Anspruch 1, wobei das Aroma L-Menthol enthält.

5. Aerosolbildende Flüssigkeit nach Anspruch 1, wobei die Hauptbestandteile Propylenglykol als das Lösungsmittel und L-Menthol als das Aroma enthalten.

## Revendications

1. Liquide générant un aérosol pour une utilisation dans un inhalateur d'aérosol, l'inhalateur d'aérosol étant configuré pour générer un aérosol par le chauffage et l'atomisation d'une solution aromatique pendant l'inhalation et pour permettre à l'aérosol d'être inhalé conjointement avec un débit d'air d'aspiration, dans lequel le liquide générant l'aérosol utilisé en tant que solution aromatique contient :
les composants principaux incluant un solvant et un arôme lipophile dissous dans le solvant ; et
de l'acide adipique ajouté aux composants principaux.

2. Liquide générant un aérosol selon la revendication 1, dans lequel l'acide adipique est présent dans une quantité de 3 % en poids par rapport aux composants principaux.

3. Liquide générant un aérosol selon la revendication 1, dans lequel le solvant contient du propylène glycol.

4. Liquide générant un aérosol selon la revendication 1, dans lequel l'arôme contient du L - menthol.

5. Liquide générant un aérosol selon la revendication 1, dans lequel les principaux composants contiennent du propylène glycol en tant que solvant et du L - menthol en tant qu'arôme.
